# EUROPEAN PATENT SPECIFICATION

(11) **EP 2 910 240 B1**
(45) Date of publication and mention of the grant of the patent: **07.07.2021**
(21) Application number: 14156581.2
(22) Date of filing: 25.02.2014
(51) Int. Cl.: A61K 9/16

(54) **Method for the manufacture of globules**
Methode zur Herstellung von Globuli
Procédé de fabrication de globule

(43) Date of publication of application: 26.08.2015
(73) Proprietor: Biologische Heilmittel Heel GmbH, 76532 Baden-Baden (DE)
(72) Inventor: Hofmann, Werner, 79639 Grenzach-Wyhlen (DE); Witt, Katharina, 76137 Karlsruhe (DE)
(74) Representative: Altmann Stößel Dick Patentanwälte PartG mbB

(56) References cited:
- WO-A1-98/07411
- US-A- 5 061 493
- US-A- 5 080 903
- MEILHAMMER B ET AL: "GLOBULI AUS HAHNEMANNS HAUSAPOTHEKE. ÖANALYTISCHE UNTERSUCHUNGEN UND VERGLEICH MIT HOMOEOPATHISCHEN GLOBULI NACH HAB", DEUTSCHE APOTHEKER ZEITUNG, DEUTSCHER APOTHEKER VERLAG, STUTTGART, DE, vol. 134, no. 17, 28 April 1994 (1994-04-28), pages 17-21, XP000441966, ISSN: 0011-9857
- None

## Description

The present invention concerns the field of drug manufacture and formulation. In particular, it relates to a method for the manufacture of drug-impregnated globules comprising the steps of providing globules to be impregnated in a rotatable vessel, rotating said vessel such that the globules move, spraying a impregnation composition comprising the drug for a time and under conditions which are sufficient to allow for a homogenous impregnation of the globules with the impregnation composition comprising the drug, spraying a washing solution to the globules for a time and under conditions which are sufficient to equilibrate the impregnation composition, and drying impregnation composition on the globules such that a homogenous impregnation is formed.

Globules are used as pharmaceutical carriers for the application of various homeopathic drugs. Globules usually consist of sugars such as sucrose, lactose, xylitol and others and have a globular shape. Typically, globules have a diameter of approx. 0.5 to 1.5 millimeters. For pharmaceutical purposes, globules are coated with, e.g., drug solutions for oral administration.

Globules are prepared according to the homeopathic Homeopathic Pharmacopeia by impregnation of the globules with a dilution of the drug and drying them. The dilution is applied by dropping (H 5.4.4, Rule 10 of the HAB (Homeopathic Pharmacopeia) 2013).

However, the dropping-impregnation of globules has some drawbacks with respect to the homogeneity of the drug impregnation formed on the globules. Thus, different globules may contain different amounts of drugs. Nevertheless, there is a need for globules which provide an essentially identical dosage of a drug to a patient.

The technical problem underlying the present invention can be seen as the provision of means and methods for complying with the aforementioned needs. The said technical problem is solved by the embodiments characterized in the claims and herein below.

Therefore, the present invention relates to a method for the manufacture of drug-impregnated globules according to claim 1. Also disclosed is a method for the manufacture of drug-impregnated globules comprising the steps of:
a) providing globules to be coated in a rotatable vessel;
b) rotating said vessel such that the globules move;
c) spraying a impregnation composition comprising the drug for a time and under conditions which are sufficient to allow for a homogenous impregnation of the globules with the impregnation composition comprising the drug;
d) spraying a washing solution to the globules for a time and under conditions which are sufficient to equilibrate the impregnation composition; and
e) drying the impregnation composition on the globules such that a homogenous impregnation is formed.

The term "globules" as used herein refers to spherical pharmaceutical carriers having a globular shape for the application of various drugs and, in particular, for homeopathic drugs. Globules preferably consist of sugars such as sucrose, lactose, xylitol and others. Typically, globules have a diameter of approx. 0.5 to 1.5 millimeters. For pharmaceutical purposes, globules are coated with, e.g., drug solutions for oral administration. For homeopathic preparations, the size of the globules depends on the potency of the drug to be used. For example, if a D or C potency is envisaged, globules of size 3 shall be used which means that 1 g of globules shall consist of approximately 110 to 130 individual globules. Details are well known to the person skilled in the art and are also found in the homeopathic pharmacopeias.

The term "drug" as used herein refers to any pharmaceutically active compound. Preferably, the drugs referred to herein are homeopathic drugs and, more preferably, herbal homeopathic drugs. Examples of such homeopathic drugs include but are not limited to excerpts from Aconitum napellus, Allium cepa, Apis mellifica, Arnica montana, Artemisia absinthium, Atropa belladonna, Avena sativa, Bellis perennis, Cetraria islandica, Coffea Arabica, Drosera, Psychotria ipecacuanha, Echinacea, Gentiana lutea, Graphites, Matricaria recutita, Hypericum perforatum, Symphytum officinalis, Thuja occidentalis, Viola tricolor or Zincum isovalerianicum. Further homeopathic drugs may be phosphorus (phosphor), silicea terra (silcium dioxide) or sulphur (sulfur).

The term "rotatable vessel" as used herein refers to any vessel which can be rotated such that globules which are inside begin to move. Preferably, such vessels may containers having a circular or oval base. Typical vessels may, therefor, be flasks, cans tons or bottles. Moreover, the vessel may need to be adapted such that the spraying can be carried out. Furthermore, the vessel may need to be suitable for applying the conditions required for spraying. Preferred conditions are referred to elsewhere herein in detail. Typically, the vessel has a single opening which allows for spraying but is otherwise closed. Such vessels can be, e.g., put into a water bath in order to adjust a certain temperature.

It will be understood that rotating of the globules in the vessel results in the movement of the globules such that all parts of the globular shape become exposed towards the spraying axis along which the coating composition is sprayed. Accordingly, the globules may exert rotational movements as well as transversal movements. Typically, the vessel is rotated with about 50 rotations per minute (rpm). Depending on the size and the geometry of the vessel the rotation parameters may need to be adjusted. How to achieve optimal rotation parameters can be determined without further ado and is well known to the person skilled in the art.

The term "about" as used in accordance with the present invention for various values of parameters means that due to measuring variations or inaccuracies, the actual value may vary by at most +/-10%, at most +/-5%, at most +/-3%, at most +/-2% or at most +/-1%. Moreover, the term about envisages also the precise indicated value, i.e. the value without any deviations.

The term "spraying" as used herein refers to applying the impregnation composition in dispersed form, e.g., as an aerosol, under pressure to the globules. Preferably, a polydisperse aerosol of the impregnation composition is generated. The dispersed impregnation composition is typically applied to the globules by a gas stream and, preferably, by an air stream.

Spraying in accordance with the present invention, preferably, comprises applying an air stream in the desired spraying direction along the spraying axis with a pressure of about 0.2 bar. Typically, a air-born-nozzle can be used for generating the air stream and for dispersing the impregnation composition to be applied to the globules therein. To this end, the impregnation composition comprising the drug is, preferably, injected into the air stream with a pressure of about 0.5 to 0.7 bar and, more preferably, about 0.6 bar. More preferably, the impregnation composition comprising the drug is injected into the air stream at a volume of about 2.4 to 2.6 ml/min and, more preferably, about 2.5 ml/min in the air-born-nozzle. The air-born-nozzle according to the present invention is, preferably, adjusted such that an oval spraying pattern or a spraying pattern with a similar geometry is obtained.

The direction of the gas stream is herein also referred to as the spraying axis because it defines the direction of the spraying. Preferably, the spraying axis in the method of the present invention is adjusted such that an ankle of about 45° is formed between the spraying axis and the bottom of the vessel containing the globules.

In accordance with the present invention it has been found that spraying shall be, preferably, applied for a time of about 17 to 23 min when using the settings described in the accompanying Examples below. It will be understood that the skilled person can adapt the spraying time depending from given settings such as vessel size etc. without further ado.

Moreover, the spraying is, typically, carried out at a temperature of about 38°C to about 42°C. Since these temperatures are above room temperature, the method of the present invention, preferably, comprises heat application of about 38°C to 42°C and, preferably, about 40°C in step c) of the method. This can be achieved by raising the temperature of the environment, e.g., by using a water bath into which the vessel may be introduced. Depending on the kind of vessel, heat application may also be achieved by heating the vessel walls directly or by heating the air in the vessel such that spraying is carried out within the aforementioned temperature range.

The term "impregnation composition" as used herein refers to a composition which shall upon application to the globules form a impregnation on the globule surface. The impregnation composition according to the present invention, preferably, comprises at least one drug and, more preferably, at least one homeopathic drug as specified elsewhere herein in detail. The impregnation composition may also comprise auxiliary components such as pharmaceutically acceptable diluents, carriers and the like. The impregnation composition shall be capable of forming a impregnation on the globules upon application. To this end, it might be necessary to remove diluents or other auxiliary components, e.g., by vaporization during drying.

The phrase "homogenous impregnation" as used in accordance with the present invention relates to a, preferably, solid layer or coat on the surface of the globules which covers essentially all parts of the surface homogenously. Since the impregnation composition may also diffuse into the surface-proximal parts of the globules, the said layer or coat may also comprise such parts of globules as far as they contain impregnation composition. The impregnation shall also be, preferably, homogeneous at all parts of the globule. Homogenous impregnation of the globules can be tested by using a dye composition as impregnation composition rather than a drug- containing composition. The dye coat obtained by applying a dye composition in the method of the invention instead a drug-containing impregnation composition can then be visually inspected for homogeneity. If necessary, parameters such as spraying pattern, rotation, air stream etc. can be adjusted until an optimal homogenous dye impregnation is obtained. Subsequently, the method can be used with a drug-containing impregnation composition.

The term "washing solution" as used herein refers to a solution which is sprayed to the globules after the impregnation composition has been applied. The washing solution shall remove excess impregnation composition from the globules. Suitable washing solutions do, thus, not react with the coating composition but can nevertheless dissolve excess material of impregnation composition (also referred to as equilibration). Typically, the washing solution applied in accordance with the method of the present invention is an alcoholic solution and, preferably, a solution comprising ethanol. More preferably, said ethanol is present in a concentration of about 62 % (w/w) in the said washing solution.

Washing in the method of the present invention is essentially carried out as described for spraying the coating composition, i.e. by spraying the washing solution onto the globules. The parameters used for spraying are essentially identical. Thus, preferably, spraying of the washing solution to the globules is carried out for about 4 min, at a temperature within the range of about 38 to 42°C and, more preferably, at about 40°C. The ankle of about 45° formed between the spraying axis and the bottom of the vessel containing the globules is, preferably, also maintained as well as the oval spraying pattern. It will be understood that, preferably, the same air-born-nozzle as for the coating composition may be used to apply the washing solutions to the globules. The pressures used for dispersing the washing solution and to generate the air stream are, preferably, the same as indicated above. Also preferably, the rotation of the vessel is maintained at this step, preferably, at 50 rpm.

The term "drying" as used herein refers to the formation of a solid impregnation from the impregnation composition. As described elsewhere herein, it might be necessary to remove certain diluents from the coating composition in order to generate a solid coat on the globules. Typically, the diluents are removed by vaporization. To this end, heat is preferably applied. More preferably, the drying step, therefore, encompasses heat application of about 38°C to 42°C and, most preferably, about 40°C. Also preferably, the rotation of the vessel is maintained at this step, preferably, at 50 rpm.

After drying of the globules, preferably, the yield is determined and the quality of the globules are visually inspected. For example, globules are investigated for agglomeration and overall appearance. Moreover, the lost of mass during drying is, preferably, determined. Further investigations, preferably, concern the flowability of the globules.

Prior to filling of the globules, globules from different batches may be mixed. For example, four batches of 5.25 kg which are the typical manufacturing size may be mixed in a 50 liters ton and rotated manually to safeguard optimal mixture of the globules of the individual batches.

A particular preferred embodiment of the method of the present invention is also described in accompanying Example 1, below.

Advantageously, it has been found in the studies underlying the present invention that the spraying of homeopathic drugs comprised in a impregnation compositions allows for a homogenous impregnation of all globules with the said impregnation composition and, thus, with essentially identical amounts of the drug. In contrast, the dropping prior art techniques produce rather in-homogenous coating. Thus, globules which are obtained by such prior art impregnation will not provide essentially identical dosages of the homeopathic drug. Thanks to the present invention, it has become possible to manufacture globules providing essentially identical amounts of drugs to the patients in need thereof. The dosage regimen can, therefore, be better adjusted for individual drugs and improper dosing shall no longer happen when using drugs impregnated on globules.

All definitions and explanations of the term made above apply mutatis mutandis to the following.

The following embodiments are particularly preferred in accordance with the method of the present invention.

In a preferred embodiment of the method of the present invention, said spraying comprises applying an air stream in the spraying direction with a pressure of about 0.2 bar.

In another preferred embodiment of the method of the present invention, the impregnation composition comprising the drug is injected into the air stream with a pressure of about 0.5 to 0.7 bar and, more preferably, about 0.6 bar. More preferably, the impregnation composition comprising the drug is injected into the air stream at a volume of about 2.4 to 2.6 ml/min and, more preferably, about 2.5 ml/min.

In a preferred embodiment of the method of the present invention, said spraying is carried out applying an oval spraying pattern.

In yet a preferred embodiment of the method of the present invention, said vessel is rotating with about 50 rpm.

In another preferred embodiment of the method of the present invention, said spraying is applied in angle of about 45° towards the bottom of the vessel.

In a further preferred embodiment of the method of the present invention, said spraying is applied for a time of about 17 to 23 min.

In a preferred embodiment of the method of the present invention, said conditions in step c) and/or d) comprise heat application of about 38°C to 42°C and, preferably, about 40°C.

In yet a preferred embodiment of the method of the present invention, said drying in step e) comprise heat application of about 38°C to 42°C and, preferably, about 40°C.

In also a preferred embodiment of the method of the present invention, said drying is carried out for a time of about 4 to 6 min and, preferably, about 5 min.

In another preferred embodiment of the method of the present invention, said drug is a homeopathic composition.

In a further preferred embodiment of the method of the present invention, said impregnation composition comprises a homeopathic composition in a pharmaceutical acceptable diluent.

In a preferred embodiment of the method of the present invention, said washing solution comprises ethanol. More preferably, said ethanol is present in a concentration of about 62 % (w/w).

The present disclosure also relates to globules having a homogenous drug- impregnation which are obtainable by the aforementioned method of the present invention.

### FIGURES

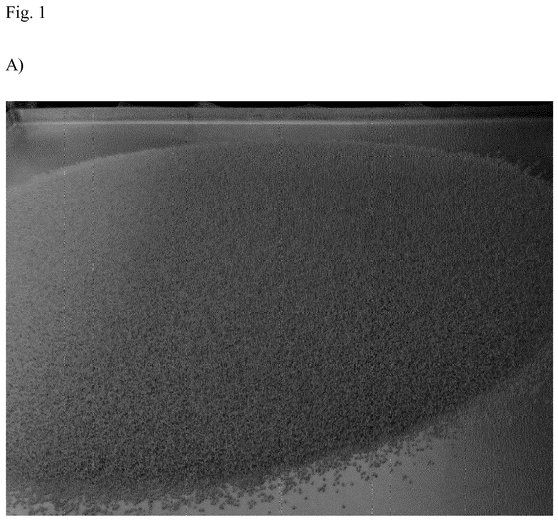

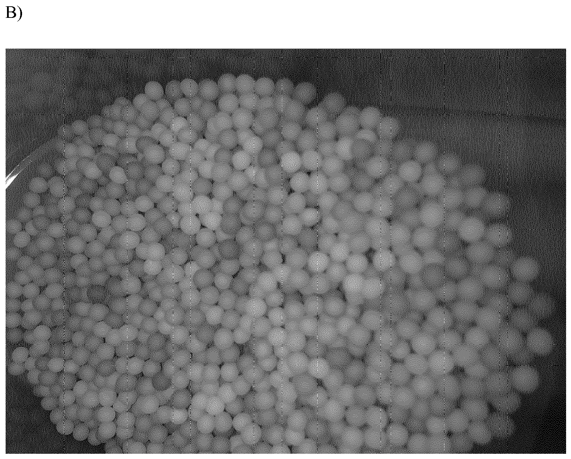

Figure 1 (A) shows a homogenous coating pattern which can be obtained by using the spraying parameters indicated in the Example 1. (B) shows in-homogenous coating obtained by the dropping method.

### EXAMPLES

### Example 1: Preparing globules by the spraying method

Using ethanol and a color paper, the spraying pattern of the air-born-nozzle connected to compressed air supply was tested with and, if necessary, adjusted such that an oval spraying pattern was obtained.

A rotary evaporator (without condenser and collecting vessel) was used with a water bath and a 20 liters laboratory flask having baffles for the globules. A two flow nozzle (breadth ca. 3 cm, height 5.5 cm, thread connection G 1/8) was introduced on a high-quality steel stick into the middle of the flask and orientated such that the spraying axis and the bottom of the flask formed a 45° ankle.

5.25 kg globules No.3 were introduced into the flask. The dosage pump was connected to a reservoir (brown glass flask) of 52.5 g coating composition ZWPR.

The aforementioned device was operated with the parameters indicated in Table 1 for spraying.

**Table 1: Operation parameters for spraying**

| Process step | Parameter |
|---|---|
| volume per time injected by the dosage pump | 2.5 ml/min |
| Spraying air stream | 0.2 bar |
| Dosage air stream | 0.6 bar |
| Temperature of the water bath | 40 °C |
| Rotation of the flask | 50 rpm |
| Orientation of the nozzle | 45° against the vessel bottom |

After about 17 to 23 min spraying, the globules are washed with a washing solution consisting of 62% (w/w) ethanol using the parameters mentioned in Table 1.

After washing has been completed, the globules were dried for 5 min in the flask at 40° C and 50 rpm.

After drying of the globules, the yield was determined and the quality of the globules was visually inspected. Further, globules were investigated for agglomeration and overall appearance. Moreover, the lost of mass during drying was determined. In addition, the flowability of the globules was investigated.

Prior to filling of the globules, globules from 4 different batches were mixed in a 50 liters ton and rotated manually to safeguard optimal mixture of the globules of the individual batches. Afterwards, globules were filled.

### Example 2: Comparison of the impregnation of globules prepared by the spraying method versus the prior art method

Impregnation of the globules manufactured according to the method described in Example 1 was compared to the impregnation of globules according to the prior art dropping method.

Globules according to the prior art were manufacture by a dropping technique carried out as follows:
A rotary evaporator (without condenser and collecting vessel) was used with a water bath and a 20 liters laboratory flask having baffles for the globules.

5.25 kg globules No.3 were introduced into the flask. 52.5 g coating composition ZWPR was applied by dropping using a pipette and allowed to impregnate for ca. 18 min at room temperature. The flask was rotated at 30 rpm. Washing was carried out using 3 ml ethanol washing solution (see Example 1) at room temperature. Drying was carried out for 20-30 min at room temperature and 50 rpm flask rotation, followed by 20-30 min at 25-30°C (in the water bath) and 50 rpm flask rotation. Drying was finished for 30-60 min at room temperature at a Horden-shelf.

After drying of the globules, the yield was determined and the quality of the globules was visually inspected. Further, globules were investigated for agglomeration and overall appearance. Moreover, the lost of mass during drying was determined. In addition, the flowability of the globules was investigated.

Prior to filling of the globules, globules from 4 different batches were mixed in a 50 liters ton and rotated manually to safeguard optimal mixture of the globules of the individual batches. Afterwards, globules were filled.

The coating of the globules obtained by the spraying method according to Example 1 and the aforementioned impregnation method (prior art) was inspected by photo-spectrometric analysis of the dye after dissolving the globules.

### Result of the comparison:

| | |
|---|---|
| Spraying method: | 90 samples consisting of 5 globules obtained from 3 independent batches (30 samples per batch) exhibited a homogenous coating deviating at most +/-15% from the average at a recovery rate of ca. 97%. |
| Dropping method | 3 samples from 10 samples consisting of 5 globules exhibited a deviation of more than +/-15% from the average and 1 sample even more than +/- 25% with respect to homogeneity of the coat. |

## Claims

1. A method for the manufacture of drug-impregnated globules comprising the steps of:
a) providing globules to be coated in a rotatable vessel;
b) rotating said vessel such that the globules move;
c) spraying a impregnation composition comprising the drug for a time and under conditions which are sufficient to allow for a homogenous impregnation of the globules with the impregnation composition comprising the drug;
d) spraying a washing solution to the globules for a time and under conditions which are sufficient to equilibrate the impregnation composition; and
e) drying coating composition on the globules such that a homogenous impregnation is formed,
wherein said drug is a homeopathic composition.

2. The method of claim 1, wherein said spraying comprises applying an air stream in the spraying direction with a pressure of about 0.2 bar.

3. The method of claim 1 or 2, wherein the impregnation composition comprising the drug is injected into the air stream with a pressure of about 0.5 to 0.7 bar and, preferably, about 0.6 bar.

4. The method of claim 3, wherein the impregnation composition comprising the drug is injected into the air stream at a volume of about 2.4 to 2.6 ml/min and, preferably, about 2.5 ml/min.

5. The method of any one of claims 1 to 4, wherein said spraying is carried out applying an oval spraying pattern.

6. The method of any one of claims 1 to 5, wherein said vessel is rotating with about 50 rpm.

7. The method of any one of claims 1 to 6, wherein said spraying is applied in angle of about 45° towards the bottom of the vessel.

8. The method of any one of claims 1 to 7, wherein said spraying in step c) is applied for a time of about 17 to 23 min.

9. The method of any one of claims 1 to 8, wherein said conditions in step c) and/or d) comprise heat application of about 38°C to 42°C and, preferably, about 40°C.

10. The method of any one of claims 1 to 9, wherein said drying in step e) comprise heat application of about 38°C to 42°C and, preferably, about 40°C.

11. The method of any one of claims 1 to 10, wherein said drying is carried out for a time of about 4 to 6 min and, preferably, about 5 min.

12. The method of any one of claims 1 to 11, wherein said impregnation composition comprises a homeopathic composition in a pharmaceutical acceptable diluent.

13. The method of any one of claims 1 to 12, wherein said washing solution comprises ethanol.

14. The method of claim 13, wherein said ethanol is present in a concentration of about 62 % (w/w).

## Patentansprüche

1. Verfahren zur Herstellung von mit Arzneimittel imprägnierten Globuli, umfassend die Schritte:
a) Bereitstellen von zu überziehenden Globuli in einem drehbaren Behälter;
b) Drehen des Behälters derart, dass sich die Globuli bewegen;
c) Sprühen einer das Arzneimittel umfassenden Imprägnierzusammensetzung für einen Zeitraum und unter Bedingungen, die für eine gleichmäßige Imprägnierung der Globuli mit der das Arzneimittel umfassenden Imprägnierzusammensetzung ausreichend sind;
d) Sprühen einer Waschlösung auf die Globuli für einen Zeitraum und unter Bedingungen, die zur Equilibrierung der Imprägnierzusammensetzung ausreichend sind; und
e) Trocknen der Überzugszusammensetzung auf den Globuli derart, dass eine gleichmäßige Imprägnierung gebildet wird,
wobei es sich bei dem Arzneimittel um eine homöopathische Zusammensetzung handelt.

2. Verfahren nach Anspruch 1, wobei beim Sprühen ein Luftstrom mit einem Druck von etwa 0,2 bar in die Sprührichtung aufgewendet wird.

3. Verfahren nach Anspruch 1 oder 2, wobei die das Arzneimittel umfassende Imprägnierzusammensetzung mit einem Druck von etwa 0,5 bis 0,7 bar und vorzugsweise etwa 0,6 bar in den Luftstrom eingespritzt wird.

4. Verfahren nach Anspruch 3, wobei die das Arzneimittel umfassende Imprägnierzusammensetzung mit einem Volumen von etwa 2,4 bis 2,6 ml/min und vorzugsweise etwa 2,5 ml/min in den Luftstrom eingespritzt wird.

5. Verfahren nach einem der Ansprüche 1 bis 4, wobei das Sprühen unter Ausführung eines ovalen Sprühmusters durchgeführt wird.

6. Verfahren nach einem der Ansprüche 1 bis 5, wobei sich der Behälter mit etwa 50 U/min dreht.

7. Verfahren nach einem der Ansprüche 1 bis 6, wobei das Sprühen in einem Winkel von etwa 45° zum Boden des Behälters ausgeführt wird.

8. Verfahren nach einem der Ansprüche 1 bis 7, wobei das Sprühen in Schritt c) für einen Zeitraum von etwa 17 bis 23 min ausgeführt wird.

9. Verfahren nach einem der Ansprüche 1 bis 8, wobei die Bedingungen in Schritt c) und/oder d) die Anwendung von Wärme von etwa 38 °C bis 42 °C und vorzugsweise etwa 40 °C umfassen.

10. Verfahren nach einem der Ansprüche 1 bis 9, wobei das Trocknen in Schritt e) die Anwendung von Wärme von etwa 38 °C bis 42 °C und vorzugsweise etwa 40 °C umfasst.

11. Verfahren nach einem der Ansprüche 1 bis 10, wobei das Trocknen für einen Zeitraum von etwa 4 bis 6 min und vorzugsweise etwa 5 min durchgeführt wird.

12. Verfahren nach einem der Ansprüche 1 bis 11, wobei die Imprägnierzusammensetzung eine homöopatische Zusammensetzung in einem pharmazeutisch unbedenklichen Verdünnungsmittel umfasst.

13. Verfahren nach einem der Ansprüche 1 bis 12, wobei die Waschlösung Ethanol umfasst.

14. Verfahren nach Anspruch 13, wobei das Ethanol in einer Konzentration von etwa 62 % (w/w) vorliegt.

## Revendications

1. Procédé pour la fabrication de globules imprégnés d'un médicament comprenant les étapes de :
a) mise à disposition de globules devant être revêtus dans une cuve rotative ;
b) rotation de ladite cuve de sorte que les globules bougent ;
c) pulvérisation d'une composition d'imprégnation comprenant le médicament pendant un temps et dans des conditions qui sont suffisants pour permettre une imprégnation homogène des globules avec la composition d'imprégnation comprenant le médicament ;
d) pulvérisation d'une solution de lavage vers les globules pendant un temps et dans des conditions qui sont suffisants pour équilibrer la composition d'imprégnation ; et
e) séchage de la composition de revêtement sur les globules de sorte qu'une imprégnation homogène soit formée,
ledit médicament étant une composition homéopathique.

2. Procédé selon la revendication 1, ladite pulvérisation comprenant l'application d'un flux d'air dans la direction de pulvérisation avec une pression d'environ 0,2 bar.

3. Procédé selon la revendication 1 ou 2, la composition d'imprégnation comprenant le médicament étant injectée dans le flux d'air à une pression d'environ 0,5 à 0,7 bar et, préférablement, d'environ 0,6 bar.

4. Procédé selon la revendication 3, la composition d'imprégnation comprenant le médicament étant injectée dans le flux d'air à raison d'un volume d'environ 2,4 à 2,6 ml/min et, préférablement, d'environ 2,5 ml/min.

5. Procédé selon l'une quelconque des revendications 1 à 4, ladite pulvérisation étant mise en œuvre avec application d'un motif de pulvérisation ovale.

6. Procédé selon l'une quelconque des revendications 1 à 5, ladite cuve tournant à environ 50 tpm.

7. Procédé selon l'une quelconque des revendications 1 à 6, ladite pulvérisation étant appliquée selon un angle d'environ 45° vers le fond de la cuve.

8. Procédé selon l'une quelconque des revendications 1 à 7, ladite pulvérisation dans l'étape c) étant appliquée pendant un temps d'environ 17 à 23 min.

9. Procédé selon l'une quelconque des revendications 1 à 8, lesdites conditions dans l'étape c) et/ou d) comprenant l'application de chaleur à une température d'environ 38 °C à 42 °C et, préférablement, d'environ 40 °C.

10. Procédé selon l'une quelconque des revendications 1 à 9, ledit séchage dans l'étape e) comprenant l'application de chaleur à une température d'environ 38 °C à 42 °C et, préférablement, d'environ 40 °C.

11. Procédé selon l'une quelconque des revendications 1 à 10, ledit séchage étant mis en œuvre pendant un temps d'environ 4 à 6 min et, préférablement, d'environ 5 min.

12. Procédé selon l'une quelconque des revendications 1 à 11, ladite composition d'imprégnation comprenant une composition homéopathique dans un diluant pharmaceutiquement acceptable.

13. Procédé selon l'une quelconque des revendications 1 à 12, ladite solution de lavage comprenant de l'éthanol.

14. Procédé selon la revendication 13, ledit éthanol étant présent en une concentration d'environ 62 % (p/p).
